# FASCICULE DE BREVET EUROPEEN

(11) **EP 1 495 075 B1**
(45) Date de publication et mention de la délivrance du brevet: **22.08.2007**
(21) Numéro de dépôt: 03740625.3
(22) Date de dépôt: 16.04.2003
(51) Int. Cl.: C08L 53/00

(54) **EMULSIONS SOLIDES A BASE D ELASTOMERE THERMOPLASTIQUE**
FESTE EMULSIONEN AUS THERMOPLASTISCHEN ELASTOMEREN
SOLID EMULSIONS BASED ON THERMOPLASTIC ELASTOMERS

(30) Priorité: 17.04.2002 FR 0204780
(43) Date de publication de la demande: 12.01.2005
(73) Titulaire: LABORATOIRES URGO, 21300 Chenove (FR)
(72) Inventeur: AUGUSTE, Stéphane, F-21490 Varois et Chaignot (FR); DESMAISON, Nadège, F-21000 Dijon (FR)
(74) Mandataire: Hubert, Philippe
(86) Numéro de dépôt international: PCT/FR2003/001216
(87) Numéro de publication internationale: WO 2003/087220

(56) Documents cités:
- WO-A-02/22735

## Description

La présente invention concerne des émulsions solides qui comprennent un élastomère thermoplastique du type copolymère poly(styrène-oléfine-styrène), une phase huileuse, une phase aqueuse et un copolymère amphiphile ainsi que leurs utilisations en particulier à des fins médicales, cosmétiques ou dermatologiques.

Il existe de très nombreuses émulsions du type eau dans huile ou huile dans eau qui ont été développées dans des applications variées comme la formulation d'explosifs, de fertilisants et plus particulièrement pour la réalisation de produits destinés à être en contact avec la peau, la plaie, les muqueuses ou les semi-muqueuses, et utilisables à des fins médicales, cosmétiques, dermatologiques ou pharmaceutiques.

Toutes ces émulsions classiques se comportent comme des produits plus ou moins visqueux ou pâteux qui, en raison de leur manque de cohésion et d'élasticité, ne peuvent être appliqués que par étalement et une fois appliqués sur la peau tendent à s'y accrocher, voire même à souiller les vêtements.

Pour remédier à ces problèmes et donner une consistance physique à ces émulsions, il a été proposé d'y incorporer, dans la phase aqueuse ou huileuse, des facteurs de consistance tels que des cires ou des gélifiants.

Mais l'addition de ces produits ne résout pas les problèmes cités précédemment de façon entièrement satisfaisante et rend plus difficile la mise au point d'émulsions stables et bien tolérées. En effet, pour stabiliser les émulsions contenant de tels facteurs de croissance, il est nécessaire d'y incorporer des quantités importantes d'un ou plusieurs agents tensioactifs, avec le risque corrélatif d'augmenter les problèmes de tolérance de ces émulsions sur la peau, la plaie ou les muqueuses.

Enfin du fait que ces émulsions ne sont ni cohésives ni élastiques une fois appliquées sur la peau, elles ne peuvent être retirées de façon réversible en une seule pièce.

On connaît par ailleurs des compositions du type gel qui sont réalisées à partir d'un copolymère séquencé poly(styrène-oléfine-styrène) associé à un plastifiant, tel une huile plastifiante comme en particulier une huile minérale.

En jouant sur la nature (grade, masse molaire) et la proportion de ces deux éléments, il est possible d'obtenir une large gamme de gels qui présentent de bonnes propriétés d'élasticité et de cohésion.

Ces compositions, qui sont utilisables dans de très nombreux domaines, ont par exemple été décrites dans les brevets US 5 167 649 et US 4 369 284.

Cependant, ces compositions connues présentent l'inconvénient d'être totalement hydrophobes en raison de la nature de leurs constituants. Il est donc impossible d'y incorporer de l'eau et il n'existe donc pas de gel de ce type contenant de l'eau.

Cette hydrophobie se traduit par un certain nombre d'inconvénients majeurs dans le cas de leur utilisation sur la peau, la plaie ou les muqueuses. En effet, il est impossible ou très difficile d'incorporer dans ces compositions connues des produits hydrophiles, comme par exemple des actifs ou des adjuvants, dont la présence peut s'avérer indispensable.

Les formulations contenant de tels actifs sont difficiles à réaliser, et nécessitent l'adjonction de composés qui en augmentent le coût de fabrication et les problèmes de tolérance vis-à-vis de la peau, la plaie ou les muqueuses.

De plus l'hydrophobie de ces gels connus conduit à la formation d'une couche occlusive qui, du fait des fluides biologiques (transpiration, exsudation de la plaie etc...) provoque des phénomènes de macération, et des risques corrélatifs de tolérance.

Enfin ces gels connus, contrairement aux hydrogels qui contiennent de l'eau, manquent d'agrément cosmétique et ne permettent pas d'apporter une sensation de froid ou de fraîcheur au contact de la peau, la plaie ou la muqueuse.

Il serait donc souhaitable de disposer de nouvelles compositions qui cumuleraient les avantages des émulsions classiques, et des compositions de type gel à base de copolymères séquences poly(styrène-oléfine-styrène) c'est à dire des compositions qui contiendraient à la fois une phase aqueuse et une phase huileuse et qui présenteraient des propriétés de cohésion et d'élasticité permettant leur manipulation, leur mise en forme, et leur retrait aisé en une seule pièce après application et qui dans le cas de leur utilisation sur la peau , la plaie ou les muqueuses seraient bien tolérées, stables, et susceptibles d'incorporer et de libérer des produits ou des actifs hydrophiles ou lipophiles, et si nécessaire, de conférer une sensation de froid ou de fraîcheur.

La présente invention a pour but de résoudre le problème technique consistant en la fourniture de nouvelles compositions répondant à ces objectifs, se présentant sous la forme d'émulsions, de type eau dans huile ou huile dans eau, élastiques et cohésives que l'on désignera dans cette demande sous le terme d'"émulsions solides".

Ainsi, selon un premier aspect, la présente demande vise à couvrir une émulsion solide caractérisée en ce qu'elle comprend :
- un élastomère thermoplastique, choisi parmi les copolymères séquencés poly(styrène-oléfine-styrène), poly(styrène-oléfine) et leurs mélanges
- une phase huileuse constituée d'un plastifiant liquide
- une phase aqueuse et
- un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère ABA pouvant être représenté schématiquement par la structure suivante : 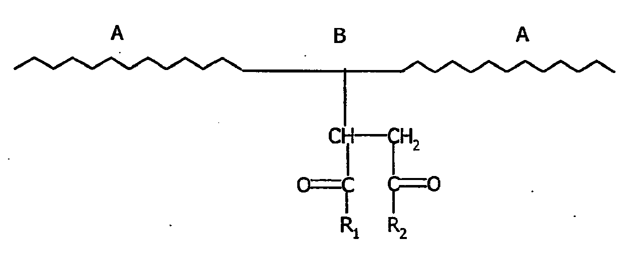
dans laquelle R₁et R₂ identiques ou différents représentent un groupement hydrophile de masse molaire moyenne inférieure à 10 000, choisi parmi les groupes suivants :

CH₃-O-(CH₂-CH₂-O)ₙ;

HO-(CH₂-CH₂-O)ₙ;

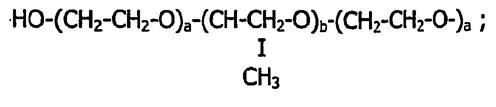

dans lesquels n, a et b représentent un nombre entier.

Ces émulsions solides présentent une excellente conformabilité et leur dureté peut être facilement réglée, ce qui permet d'obtenir des produits allant du gel cohésif au solide, utilisables non seulement dans de multiples domaines d'applications, tout comme les gels connus à base de copolymères séquencés, mais également dans des applications où ils peuvent être mis en contact avec la peau , la plaie ou les muqueuses.

Selon un second aspect, la présente demande vise à couvrir l'utilisation de ces nouvelles émulsions solides pour la réalisation de produits destinés à des fins médicales, dermatologiques ou cosmétologiques et susceptibles de venir au contact de la plaie, de la peau ou des muqueuses.

La description détaillée qui va suivre des différents constituants des émulsions solides selon l'invention, permettra de mieux comprendre la nature et les applications de cette invention.

### Description de l'invention

Le copolymère amphiphile utilisé dans la réalisation des émulsions solides selon l'invention est un copolymère à blocs du type ABA comportant deux blocs terminaux thermoplastiques A poly(styrène) et un bloc central élastomère B dans lequel ce bloc central B est une séquence poly(éthylène-butylène) comportant des groupes hydrophiles greffés, ledit copolymère amphiphile ABA pouvant être représenté schématiquement par la structure suivante : 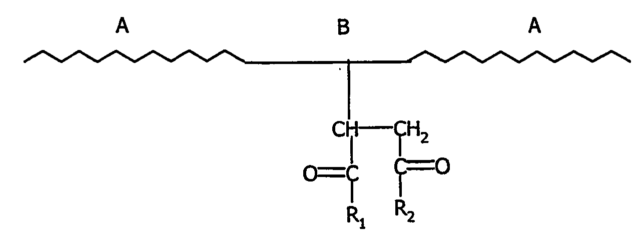 dans laquelle R₁ et R₂ identiques ou différents représentent un groupement hydrophile de masse molaire moyenne inférieure à 10 000, choisi parmi les groupes suivants :

CH₃-O-(CH₂-CH₂-O)ₙ ;

HO-(CH₂-CH₂-O)ₙ ;

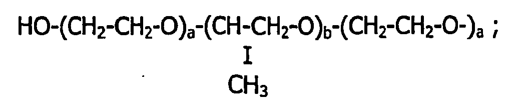

dans lesquels n, a et b représentent un nombre entier.

Avantageusement, dans le cadre de la présente invention, on préférera les copolymères amphiphiles dans lesquels R₁ et R₂ sont identiques.

Parmi ceux-ci, on utilisera de préférence les copolymères amphiphiles dans lesquels R₁ et R₂ représentent un groupe CH₃-O-(CH₂-CH₂-O)ₙ, en particulier de masse molaire moyenne comprise entre 1000 et 8000 et tout particulièrement de masse molaire moyenne égale à 2000 (soit n=45).

Ces copolymères amphiphiles sont obtenus par greffage de composés hydrophiles sur un copolymère SEBS particulier.

Ce copolymère particulier comporte des fonctions anhydrides succiniques réparties le long de la chaîne élastomérique poly(éthylène-butylène), obtenues par réaction de l'anhydride maléique sur la séquence poly(éthylène-butylène) et on l'appellera par la suite SEBS maléisé.

Ce copolymère SEBS maléisé, qui sert de base à la réalisation des copolymères amphiphiles selon l'invention, peut être représenté schématiquement par la formule suivante :

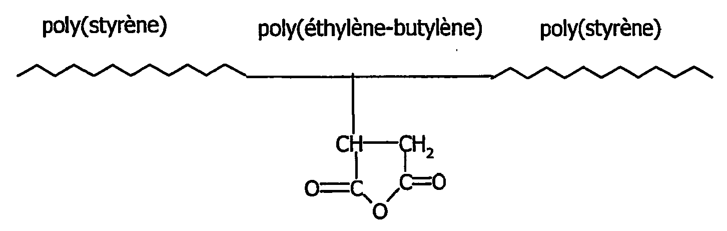

Dans cette formule, on n'a représenté, pour des raisons de simplicité, qu'un seul groupe anhydride succinique sur la séquence poly(éthylène-butylène). Il est bien évident que cette séquence comporte en réalité plusieurs groupes anhydriques succiniques. Cette simplification a également été utilisée pour représenter schématiquement les copolymères amphiphiles utilisés dans le cadre de la présente invention.

En tant que SEBS maléisé on préférera un SEBS maléisé commercialisé par la société SHELL sous la dénomination Kraton G 1901® qui contient 2 % en poids de fonctions anhydrides succiniques fixées sur la chaîne élastomérique et 28 % en poids de polystyrène.

Ce sont ces fonctions anhydrides qui vont servir à greffer les composés hydrophiles par réaction chimique entre le composé hydrophile et l'anhydride ou sa forme acide.

En effet, selon les conditions de stockage et en particulier suivant le degré de séchage de ce SEBS maléisé une partie de ces fonctions anhydrides succiniques peuvent se présenter sous leurs formes acide après ouverture de l'anhydride en présence d'eau. La réaction s'effectue alors aussi entre les fonctions acides et le composé hydrophile.

Les composés hydrophiles qui sont greffés sur le SEBS maléisé sont de 3 types :

### A/ Les polyéthylèneglycols appelés ci-après en abrégé PEG

Ce sont des polymères hydrophiles, hygroscopiques et stables thermiquement. Ils sont utilisés dans de très nombreux domaines industriels. Ils sont bien connus de l'homme de l'art. Ce sont des polymères de courtes chaînes possédant des fonctions hydroxyles aux extrémités. Leur masse molaire moyenne varie de 200 à 20 000.

Leur composition correspond à la structure suivante :
HO-(CH₂-CH₂-O)ₙ-H, dans laquelle n représente un nombre entier.

De tels produits sont par exemple commercialisés par la société Aldrich sous la dénomination poly(éthylèneglycol) suivie de la masse molaire moyenne du PEG considéré, par exemple poly(éthylèneglycol) 2000.

Dans le cadre de la présente invention seuls des copolymères amphiphiles dans lesquels les PEG de masse molaire moyenne inférieure ou égale à environ 10 000 (n ayant donc au maximum une valeur de 230) sont utilisés. En effet, au-delà la réaction de greffage devient difficile voire impossible.

Avantageusement, on utilisera les PEG qui ont une masse molaire moyenne comprise entre 1 000 et 8 000 et en particulier le PEG qui a une masse molaire moyenne de 2 000 (n = 45).

### B/ Les polyéthylèneglycols mono méthyl éther appelés ci-après en abrégé PEGME

Ce sont aussi des polymères de courtes chaînes utilisés comme les PEG dans de très nombreux domaines et bien connus de l'homme de l'art.

Ils ont la structure suivante :
CH₃-O-(CH₂-CH₂-O)ₙ-H, dans laquelle n est un nombre entier, et leur masse molaire moyenne varie de 200 à 20 000.

De tels produits sont par exemple commercialisés par la société Aldrich sous la dénomination poly(éthylèneglycol)méthyl éther suivie de la masse molaire moyenne du PEGME considéré, par exemple poly(éthylèneglycol)méthyléther 2000.

Dans le cadre de la présente invention on utilise des copolymères amphiphiles dans lesquels tout comme pour les PEG, seuls les PEGME de masse molaire moyenne inférieure ou égale à environ 10 000 (n ayant au maximum une valeur de 230) sont utilisés.

Avantageusement, on utilisera les PEGME qui ont une masse molaire moyenne comprise entre 1 000 et 8 000 et en particulier le PEGME qui a une masse molaire moyenne de 2 000 (n = 45).

### C/ Les copolymères de polyéthylène-polypropylèneglycol

Ce sont des copolymères très connus que l'on désignera ci-après en abrégé PEO/PPO/PEO.

Ce sont des copolymères triblocs dont la partie centrale est un bloc oxyde de polypropylène et les extrémités des blocs oxyde de polyéthylène qui ont la structure suivante : 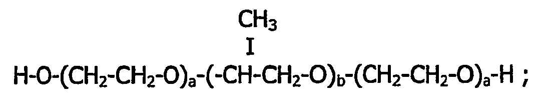 dans laquelle a et b sont des nombres entiers.

Ils sont souvent désignés sous le terme général de poloxamer.

Il existe pour ces produits de très nombreux grades caractérisés par les valeurs de a et b qui définissent leurs masses molaires moyennes. On peut ainsi citer :
- poloxamer 124 : a=12 b=20 masse molaire moyenne comprise entre 2 090 et 2360
- poloxamer 188 : a=80 b=27 masse molaire moyenne comprise entre 7 680 et 9510
- poloxamer 407 : a=101 b=56 masse molaire moyenne comprise entre 9 840 et 14 600.

Ils sont commercialisés par exemple par la société BASF sous la dénomination Pluronic®.

Ici aussi comme précédemment seuls les PEO/PPO/PEO de masse molaire moyenne inférieure ou égale à environ 10 000 seront utilisés.

Dans le cadre de la présente invention on préférera un PEO/PPO/PEO de masse molaire voisine de 2000 comme par exemple le produit commercialisé sous la dénomination poly(éthylèneglycol)-block-poly(propylèneglycol)-block-poly(éthylèneglycol) 1900 par la société Aldrich, de masse molaire moyenne égale à 1900.

Les copolymères amphiphiles utilisables dans le cadre de la présente invention peuvent être facilement préparés par réaction d'estérification entre les fonctions anhydrides succiniques du SEBS maléisé et les fonctions hydroxyles des PEG, PEGME ou PEO/PPO/PEO utilisés.

La réaction d'un alcool sur une fonction anhydride donne de façon réversible un ester. Dans le cadre de la présente invention, cette estérification peut être représentée par le schéma simplifié suivant :

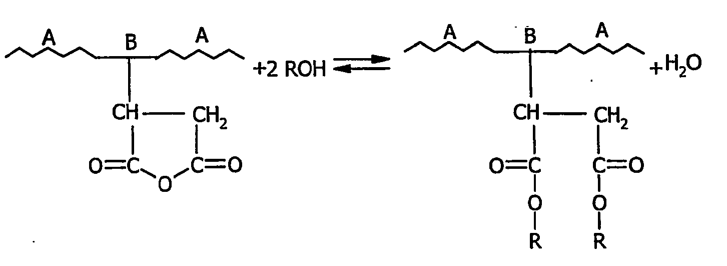

Afin de favoriser la réaction d'estérification on introduit un excès de fonctions hydroxyles par rapport aux fonctions anhydrides. La réaction est avantageusement catalysée par un acide, et l'eau formée est éliminée par distillation azéotropique pour déplacer l'équilibre vers le produit greffé. La réaction est réalisée de préférence sous atmosphère inerte.

Ainsi on prépare lesdits copolymères amphiphiles selon un procédé dans lequel on réalise une réaction d'estérification entre les fonctions anhydrides succiniques portées par la partie poly(éthylène-butylène) d'un copolymère poly(styrène)-poly(éthylène-butylène)-poly(styrène) (SEBS maléisé) et les fonctions hydroxyles d'un composé hydrophile choisi parmi les polyéthylèneglycols, (PEG), les polyéthylèneglycols mono méthyléther (PEGME) et les copolymères de polyéthylène-propylène glycol (PEO/PPO/PEO) de masse molaire moyenne inférieure ou égale à 10 000 ou leurs mélanges, de préférence en présence d'un catalyseur acide, en éliminant l'eau formée et avec un excès de fonctions hydroxyles par rapport aux fonctions anhydrides succiniques du SEBS maléisé.

De façon plus précise le procédé de synthèse est le suivant :
On dissout à chaud (à environ 120°C température de reflux du solvant) et sous agitation le SEBS maléisé dans un solvant, de préférence le toluène.
On prépare à part une solution d'au moins un composé hydrophile (PEG, PEGME, PEO/PPO/PEO ou leurs mélanges) en chauffant ce(s) dernier(s) à sa(leur) température de fusion sous agitation dans un solvant, de préférence le toluène. On utilise avantageusement un excès de composés hydrophiles. Le nombre de fonctions hydroxyles rapporté au nombre de fonctions anhydrides peut ainsi varier de 2,5 à 20.
A la solution de copolymère SEBS maléisé préalablement obtenue sous agitation et toujours à reflux, on ajoute une quantité catalytique (environ quelques gouttes) d'acide, par exemple d'acide sulfurique, puis la solution de composé(s) hydrophile(s) dans le solvant préparée précédemment.
On agite sous distillation azéotropique ce mélange à reflux durant 30 minutes à 5 heures selon la nature du(des) composé(s) hydrophile(s) jusqu'à réalisation complète de la réaction d'estérification entre les fonctions anhydrides (ou leurs éventuelles formes acides) des groupes succiniques du SEBS maléisé et les fonctions hydroxyles du(des) composé(s) hydrophile(s). L'état d'avancement de la réaction est suivi en utilisant les techniques bien connues de l'homme de l'art, par exemple par spectroscopie infra rouge jusqu'à disparition du pic d'absorption des carbonyls de l'anhydride soit 1785 cm⁻¹.
On précipite alors le mélange réactionnel à chaud à environ 90-100 °C dans un solvant de précipitation adéquat comme par exemple l'éthanol ou un mélange éthanol/eau, ledit solvant de précipitation représentant environ 4 fois le volume de l'ensemble des solvants réactionnels utilisés.
Après filtration, les solvants résiduels sont éliminés du copolymère SEBS amphiphile obtenu, par évaporation à l'étuve sous vide à 40-50 °C.
Il est alors nécessaire de purifier ce dernier pour éliminer le(s) composé(s) hydrophile(s) PEG, PEGME ou PEO/PPO/PEO utilisé(s) en excès encore présent(s).
On redissout donc le polymère amphiphile obtenu sous agitation à environ 90 à 110°C dans le toluène et on reprécipite la solution obtenue dans le même solvant et le même volume que lors de l'étape de précipitation réalisée précédemment à la fin de la synthèse.
De même le copolymère SEBS amphiphile est récupéré par filtration et séché à nouveau à l'étuve sous vide à 40-50°C.

On répète cette étape de purification jusqu'à élimination totale du(des) composé(s) hydrophile(s) en vérifiant selon les techniques connues de l'homme de l'art l'absence du pic de ce(ces) dernier(s) par chromatographie par perméation de gel (GPC).

Dans le cadre de la présente invention on utilisera ce copolymère amphiphile dans les émulsions solides à une concentration de l'ordré de 0,05 % à 20 % en poids par rapport au poids total de la composition.

Selon un mode de réalisation préférée de l'invention on utilisera un copolymère amphiphile à blocs du type ABA comportant deux blocs terminaux thermoplastiques A poly(styrène) et un bloc central élastomère B dans lequel ce bloc central B est une séquence poly(éthylène-butylène) comportant des groupes hydrophiles greffés, ledit copolymère amphiphile ABA pouvant être représenté par la structure suivante 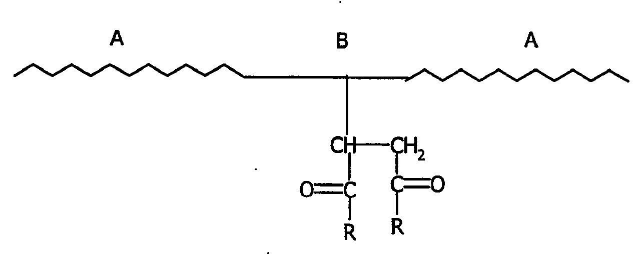 dans laquelle R représente un groupe CH₃-O-(CH₂-CH₂-O)ₙ de masse molaire moyenne égale à 2000 soit n=45.

On préférera tout particulièrement un tel copolymère amphiphile qui a une masse molaire moyenne, mesurée par chromatographie par perméation de gel, de l'ordre de 50 000 daltons.

De préférence, on utilisera alors ce dernier dans les émulsions solides à une concentration de l'ordre de 0,05 à 20% et en particulier de 0,05 à 5% en poids par rapport au poids total de la composition.

Les élastomères thermoplastiques du type copolymères séquencés (styrène-oléfine-styrène) ou copolymères séquencés (styrène-oléfine) qui sont susceptibles d'être utilisés dans le cadre de la présente invention sont ceux habituellement utilisés par l'homme de l'art dans la préparation de compositions comme par exemple des adhésifs sensibles à la pression et l'on pourra se reporter à cet égard à l'ouvrage édité en 1989 par Donatas Satas « Handbook of Pressure Sensitive Technology », 2ème édition, chapitre 13, pages 317 à 359, aux demandes de brevet Européen publiées sous les numéros EP 758 009 ou EP 723 571 ou aux documents de l'état de la technique mentionnés précédemment.

Ce sont donc soit des copolymères triblocs du type ABA comportant deux blocs terminaux thermoplastiques A styrène et une séquence centrale élastomère B qui est une oléfine, soit des copolymères diblocs du type AB comportant un bloc thermoplastique A styrène et une séquence élastomère B qui est une oléfine. Les séquences B oléfines de ces copolymères peuvent être constituées d'oléfines insaturées comme par exemple isoprène ou butadiène ou d'oléfines saturées comme par exemple éthylène-butylène ou éthylène-propylène.

On pourra, dans le cadre de la présente invention, utiliser l'ensemble de ces produits seuls ou en mélange.

Dans le cas d'un mélange de copolymères triblocs ABA et de copolymères diblocs AB, on pourra employer des mélanges de copolymères triblocs ABA et de copolymères diblocs AB commerciaux déjà disponibles ou réaliser un mélange en toute proportion préalablement choisie à partir de deux produits indépendamment.

Les produits à séquence centrale insaturée sont bien connus de l'homme de l'art et sont par exemple commercialisés par la société SHELL sous la dénomination KRATON^{®} D. On peut aussi citer pour les copolymères poly(styrène-isoprène-styrène) (en abrégé SIS) les produits commercialisés sous les dénominations KRATON® D 1107 ou KRATON® D 1161 et pour les copolymères poly(styrène-butadiène-styrène) par exemple le produit commercialisé sous la dénomination KRATON® D 1102. D'autres copolymères poly(styrène-isoprène-styrène) sont aussi commercialisés par la société EXXON CHEMICAL sous la dénomination VECTOR® comme par exemple le produit commercialisé sous la dénomination VECTOR® 4113. Comme exemple d'un mélange commercial de copolymères triblocs ABA et diblocs AB, on peut citer le produit commercialisé par la société EXXON CHEMICAL sous la dénomination VECTOR® 4114 dans lequel B est de l'isoprène.

Tous ces copolymères à base d'isoprène ou de butadiène présentent généralement une teneur en styrène comprise entre 10 et 52% en poids par rapport au poids total dudit copolymère.

Dans le cadre de la présente invention, on préférera les copolymères séquences triblocs poly(styrène-isoprène-styrène) ayant une teneur en styrène comprise entre 14 et 30% en poids par rapport au poids dudit SIS et en particulier le produit commercialisé par la société SHELL sous la dénomination KRATON® D 1161.

Les produits à séquence centrale saturée sont aussi bien connus de l'homme de l'art et sont par exemple commercialisés par la société SHELL sous la dénomination KRATON® G pour les copolymères séquencés poly(styrèneéthylène-butylène-styrèné) (en abrégé SEBS) comme par exemple les produits commercialisés sous les dénominations KRATON® G 1651 ou KRATON® G 1654 ou par la société KURARAY sous la dénomination SEPTON® pour les copolymères séquences poly(styrène-éthylène-propylène-styrène) (en abrégé SEPS).

Comme exemple de mélanges commerciaux de copolymères tribloc-dibloc on peut citer le produit dont la séquence oléfine est éthylène-butylène commercialisé par la société SHELL sous la dénomination KRATON® G 1657.

Comme exemple d'un mélange particulier tribloc-dibloc, que l'on peut utiliser dans le cadre de la présente invention, on peut citer le mélange d'un SEBS tribloc comme le produit commercialisé par la société SHELL sous la dénomination KRATON® G 1651 avec un matériau dibloc poly(styrène-oléfine) comme le poly(styrène-éthylène-propylène) commercialisé par la société SHELL sous la dénomination KRATON® G 1702.

Dans le cadre de la présente invention, on préférera les copolymères triblocs SEBS ou SEPS et en particulier ceux ayant une teneur en styrène comprise entre 25 et 45% en poids par rapport au poids dudit SEBS. On préférera tout particulièrement le produit commercialisé par la société SHELL sous la dénomination KRATON® G 1651.

De façon générale, on utilisera l'élastomère thermoplastique selon la nature du copolymère séquencé, en une quantité de l'ordre de 2 à 20% en poids par rapport au poids total de la composition. De préférence, on utilisera un élastomère thermoplastique qui a une masse molaire moyenne supérieure à celle du copolymère amphiphile et de préférence de l'ordre de 100 000 daltons. Dans ce cas, on utilisera alors ce dernier de préférence, en une quantité de l'ordre de 2 à 10% en poids par rapport au poids total de la composition.

Si nécessaire, on pourra ajouter à ces copolymères séquencés des agents antioxydants.Par agents antioxydants, on entend désigner ici les composés couramment employés par l'homme de l'art pour assurer la stabilité vis-à-vis de l'oxygène, la chaleur, l'ozone et les rayonnements ultra violets des composés utilisés dans les formulations à base d'élastomère thermoplastique. On peut utiliser un ou plusieurs de ces agents antioxydants en association.

On peut citer comme agents antioxydants appropriés les antioxydants phénoliques comme par exemple les produits commercialisés par la société CIBA-GEIGY sous les dénominations IRGANOX^{®} 1010, IRGANOX^{®} 565, IRGANOX^{®} 1076 et des antioxydants soufrés comme par exemple le dibutyldithiocarbamate de zinc commercialisé par la société AKZO sous la dénomination PERKACIT® ZDBC.

Dans les émulsions solides selon la présente invention, la phase huileuse est une phase hydrophobe et lipophile qui est constituée par un plastifiant liquide. On désigne par "plastifiant liquide", les plastifiants qui sont habituellement utilisés par l'homme de l'art pour la préparation de compositions comprenant des élastomères thermoplastiques en particulier du type copolymères séquencés poly(styrène-oléfine-styrène) comme par exemple des adhésifs thermofusibles sensibles à la pression et qui sont des produits qui permettent d'améliorer leurs propriétés d'étirement, de souplesse, d'extrudabilité ou de mise en oeuvre et l'on pourra se reporter à cet égard aux documents de l'état de la technique mentionnés précédemment.

Ces plastifiants liquides sont des composés compatibles avec la séquence centrale oléfine des copolymères séquences utilisés. On utilise très souvent comme plastifiant liquide des huiles plastifiantes, et en particulier des huiles minérales qui sont formées de composés de nature paraffinique, naphténique ou aromatique ou de leurs mélanges dans des proportions variables.

On peut ainsi citer comme exemple d'huiles minérales les produits commercialisés par la société SHELL sous la dénomination ONDINA^{®} et RISELLA^{®} pour les mélanges à base de composés naphténiques et paraffiniques ou sous la dénomination CATENEX® pour les mélanges à base de composés naphténiques, aromatiques et paraffiniques.

Dans le cadre de la présente invention on préférera les huiles de paraffine et en particulier l'huile commercialisée par la société SHELL sous la dénomination ONDINA^{®} 15.

On peut aussi utiliser comme plastifiant liquide non pas une huile plastifiante mais des produits de synthèse à base de mélanges liquides d'hydrocarbures saturés comme par exemple les produits commercialisés par la société TOTAL sous la dénomination GEMSEAL comme en particulier le produit GEMSEAL^{®} 60 qui est un mélange isoparaffinique issu d'une coupe pétrolière totalement hydrogénée.

Dans le cadre de la réalisation d'émulsions solides selon l'invention, on utilisera de préférence une concentration en plastifiant liquide de l'ordre de 25 à 90% en poids par rapport au poids total- de l'émulsion solide et de préférence de 30 à 75% en poids par rapport au poids total de l'émulsion solide.

La phase aqueuse des émulsions solides selon l'invention est principalement de l'eau. On pourra utiliser tout type d'eau suivant les domaines d'applications envisagés comme par exemple de l'eau de source, de l'eau du robinet, de l'eau déminéralisée, purifiée, déionisée ou stérilisée.
On pourra bien entendu incorporer avec cette eau tout adjuvant utile pour conserver au cours du temps ces propriétés de pureté ou de stérilité ou tous produits utilisés généralement dans la réalisation d'émulsion classique qui sont incorporés dans la phase aqueuse. Bien entendu, l'homme du métier veillera à ce que l'introduction ou les proportions de ces produits ne viennent pas altérer les propriétés de cohésion et d'élasticité des émulsions solides obtenues.

De même suivant l'application envisagée on pourra introduire de très faibles quantités d'eau de l'ordre de 1% en poids par rapport au poids total de l'émulsion ou de grandes quantités allant jusqu'à 60% ou plus en poids par rapport au poids total de l'émulsion solide.

Dans le cadre de la présente invention on préfère des émulsions solides qui comprennent de l'ordre de 5 à 60% en poids d'eau et tout particulièrement de 10 à 50 % en poids par rapport au poids total de l'émulsion solide.

Une émulsion solide actuellement préférée selon l'invention comprend :
a. 2 à 10 parties en poids d'un élastomère thermoplastique de masse molaire moyenne supérieure ou égale à 100 000 daltons
b. 30 à 75 parties en poids de plastifiant liquide
c. 2 à 50 parties en poids d'eau et
d. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 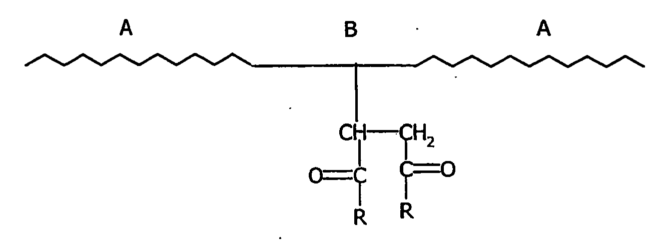
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

Tout comme les émulsions classiques et les gels à base de copolymères séquencés précédemment décrits, les émulsions solides selon l'invention peuvent être utilisées dans de multiples domaines grâce à leur excellente capacité de mise en forme et à leur caractère hydrophile qui peut être facilement réglé en fonction de l'utilisation envisagée.

En effet, les émulsions solides selon l'invention sont des produits thermofusibles qui sont fabriqués en fondant et mélangeant les constituants à la température de fusion de l'élastomère thermofusible utilisé selon la technique bien connue de l'homme de l'art désignée sous le nom de procédé « holt melt » . Grâce aux propriétés rhéologiques des émulsions solides selon l'invention, le mélange homogène obtenu en final peut donc être mis en forme ou combiné avec d'autres éléments, avant refroidissement, pour réaliser un produit final.

On peut ainsi réaliser des produits finis sous diverses formes géométriques par moulage ou extrusion comme par exemple des plaques conformables d'épaisseur variables, des spaghettis, des films, des granulés. On peut aussi déposer l'émulsion solide chaude sur un élément formant support, par exemple par enduction.

Toutes ces possibilités offrent de très nombreux avantages et s'avèrent particulièrement utiles dans la réalisation de produits à destinations médicales, dermatologiques, pharmaceutiques ou cosmétiques. Les émulsions solides selon l'invention pourront être facilement incorporées dans tout dispositif destiné à être mis en contact avec la peau, la plaie ou les muqueuses, par exemple un pansement ou un bandage pour le traitement ou la protection de la peau, de la plaie, des brûlures, de l'ampoule, des lésions dermoépidermiques superficielles, profondes, chroniques ou aiguës ; un patch pour la libération topique ou systémique d'actifs ; un produit pour le nettoyage ou le soin de la peau, comme par exemple un produit désincrustant ou anti-rides ; ou encore une électrode.

Les émulsions solides selon l'invention peuvent être également utilisées seules, en cosmétologie ou dermatologie pour réaliser des gels ou des pommades que l'on peut retirer d'une seule pièce ou dans le domaine du traitement de brûlures, pour réaliser des plaques de recouvrement conformables, ou encore en chirurgie pour réaliser un matériau biocompatible implantable, par exemple un renfort de paroi abdominale,

De plus, grâce à leur aptitude à contenir de l'eau ou un actif hydrophile dans un milieu à forte teneur hydrophobe et vice versa, divers composés peuvent être incorporés lors de la formulation de l'émulsion solide. Ces composés peuvent être des adjuvants ou des actifs couramment utilisés dans les domaines dermatologiques, cosmétiques ou pharmacologiques. En tant qu'adjuvants susceptibles d'être ainsi incorporés, on peut citer des antioxydants, des conservateurs, des parfums, des charges, des absorbeurs d'odeurs, des matières colorantes, des filtres UV, des électrolytes pour conduire le courant, des régulateurs de pH, des bactéricides, des particules magnétisables, des microcapsules ou des microsphères.

Les quantités de ces différents adjuvants sont celles classiquement utilisées dans le domaine considéré et par exemple de 0,01 à 20% en poids par rapport au poids total de l'émulsion. Comme agent actif, on peut incorporer un ou plusieurs actifs choisis parmi la liste suivante : vitamines et leurs dérivés, glycérine, collagène, acide salicylique, huiles essentielles aromatiques, caféine, actifs anti-radicaux libres, hydratants, dépigmentants (tel l'acide Kojique), liporégulateurs, anti-acnéiques, anti-vieillissement, adoucissants, décongestionnants, antirides, rafraîchissants, agents kératolitiques et agents accélérateurs de cicatrisation, protecteurs vasculaires, anti-bactériens tel la sulfadiazine argentique, antifongiques, anti-perspirants, déodorants, agents conditionneurs de la peau, composés insensibilisants, immunomodulateurs et nourrissants, extraits végétaux comme par exemple le thé vert, l'arnica, l'hamamélis, oligo-éléments, anesthésiques locaux, anti-inflammatoires, hormones, menthol, rétinoïdes, la DHEA, les extraits d'algues, de champignons, de levure, de bactéries, les protéines hydrolysées, partiellement hydrolysées ou non ou les enzymes.

Le ou les actifs peuvent être par exemple présents en une concentration allant de 0,01 à 20%, de préférence de 0,1 à 5%, et mieux de 0,5 à 3% du poids total de l'émulsion.

Ces adjuvants ou ces actifs, selon leur nature, peuvent être introduits dans la phase hydrophobe et lipophile ou dans la phase aqueuse. Bien entendu, l'homme du métier veillera à choisir les éventuels actifs ou adjuvants complémentaires et/ou leur quantité de telle manière que les propriétés avantageuses de cohésion, et d'élasticité de l'émulsion solide selon l'invention ne soient pas ou substantiellement pas altérées par l'adjonction envisagée.

Dans le cadre de la réalisation de produits à des fins médicales, dermatologiques ou cosmétiques comme par exemple des patchs, des pansements, des électrodes, l'émulsion solide qui sert de réservoir d'actifs ou d'adjuvants est en général associée à un support.

Etant donné que l'émulsion solide est thermofusible, on réalise dans ce cas l'enduction de l'émulsion sur un support adéquat au grammage souhaité, selon la technique bien connue de l'homme de l'art désignée sous le nom de procédé holt melt. Le produit final pourra être fixé sur la peau, la plaie ou les muqueuses à l'aide si nécessaire d'un adhésif par exemple périphérique.

Le choix du support est réalisé en fonction des propriétés requises (étanchéité, élasticité suivant le type de produit et l'application recherchée.

Il peut se présenter sous forme d'un film d'une épaisseur variable de 5 à 150 µm ou d'un non-tissé ou d'une mousse ayant une épaisseur de 10 à 500 µm. Ces supports à base de matériaux synthétiques ou naturels sont ceux généralement utilisés par l'homme de l'art dans le domaine des applications visées ci-dessus.

On peut citer ainsi des mousses en polyéthylène, en polyuréthanne, en PVC, des non-tissés en polypropylène, polyamide, polyester ou des complexes réalisés à base d'un film et d'un non-tissé

D'une façon pratique, la surface de l'émulsion solide qui n'est pas liée au support, pourra être recouverte d'une couche ou pellicule de protection pelable avant utilisation du produit. L'ensemble ainsi formé pourra être lui-même emballé dans une protection étanche réalisée par exemple au moyen de complexes polyéthylène-aluminium ou dans des blisters.

Les caractéristiques et applications de l'invention seront mieux comprises à la lecture de la description qui va suivre d'exemples de réalisation. Bien entendu, l'ensemble de ces éléments n'est nullement limitatif mais est donné uniquement à titre d'illustration.

Pour des raisons de simplicité, on donnera tout d'abord un exemple de synthèse d'un copolymère amphiphile représentatif qui sera utilisé dans toutes les compositions adhésives données à titre d'exemples par la suite.

Le procédé de synthèse de ce copolymère est décrit dans la préparation I ci-après.

Pour réaliser la synthèse de ce dernier, on utilise un réacteur muni d'un réfrigérant, équipé d'un desséchant, d'un sas relié au vide et à l'azote si la réaction est effectuée sous atmosphère inerte, et d'un Dean Stark pour éliminer l'eau formée par distillation azéotropique.

### PREPARATION I

Sous azote on introduit dans le réacteur 150 ml de toluène et on ajoute 20 g de Kraton G 1901® (copolymère SEBS maléisé) commercialisé par la société SHELL. On chauffe à reflux (environ 110°C) sous agitation jusqu'à dissolution totale du copolymère SEBS maléisé. On prépare à part une solution de PEGME de masse molaire 2 000 commercialisé par la société Aldrich sous la dénomination poly(éthylèneglycol)méthyléther 2000. On dissout ainsi sous agitation 32,32 g de PEGME 2000 en chauffant à sa température de fusion dans 100 ml de toluène. A la solution de copolymère SEBS maléisé préalablement obtenue, on ajoute toujours sous agitation et à reflux, environ 20 gouttes d'acide sulfurique. On _ ajoute ensuite toujours sous agitation et à reflux la solution de PEGME 2000 dans le toluène préparée précédemment. On a ainsi dans ce cas 4 fonctions hydroxyles pour chaque fonction anhydride. On agite toujours à reflux le mélange obtenu jusqu'à réalisation complète de la réaction d'estérification, soit ici environ 30 à 40 minutes. On précipite alors à chaud, à environ 90 à 100°C, la solution dans 1,5 litre d'un mélange eau-éthanol 50/50. Après filtration les solvants résiduels sont éliminés du précipité obtenu par évaporation à l'étuve sous vide à 40-50°C. Pour purifier le polymère amphiphile obtenu il est nécessaire d'éliminer le PEGME 2000 en excès qui n'a pas réagi lors de la synthèse. Pour cela, on redissout à chaud aux environs de 90-100 °C, en agitant, le polymère amphiphile dans 100 à 150 ml de toluène et la solution obtenue est à nouveau précipitée dans 1,5 litre d'un mélange eau-éthanol 50/50. Après filtration, le précipité récupéré est séché sous vide à 40-50°C. On répète cette étape de purification (redissolution-précipitation et séchage sous vide) jusqu'à élimination totale du PEGME 2000. On obtient ainsi un copolymère amphiphile dans lequel R₁ et R₂ représentent un groupe CH₃-O-(CH₂-CH₂-O-)ₙ dans lequel n=45 qui a une masse molaire moyenne mesurée par chromatographie par perméation de gel, avec comme solvant du tétrahydrofuranne à un débit de 1 ml par minute, sur une colonne commercialisée par la société WATERS sous la dénomination STYRAGEL HR4 et avec un détecteur à indice de réfraction, de l'ordre de 50 000 daltons.

### EXEMPLES D'EMULSIONS SOLIDES SELON L'INVENTION

On a alors réalisé plusieurs émulsions solides selon l'invention selon le procédé de fabrication suivant :
Le mélange des différents constituants est réalisé dans un réacteur fermé, chauffé à une température qui varie entre 100 et 130°C, selon la nature de l'élastomère thermoplastique, de manière à fondre ce dernier.
L'agitation est effectuée grâce à un mélange muni d'une hélice défloculeuse à une vitesse d'environ 500 tours par minute.

Dans un premier temps, on introduit dans le réacteur chauffé entre environ 100 et 130°C le copolymère amphiphile, l'eau, le plastifiant liquide et

tous les autres composés, actifs ou adjuvants, non susceptibles de se dégrader à cette température hormis l'élastomère thermoplastique, puis on agite le mélange ainsi formé de façon continue jusqu'à obtenir un mélange homogène.

Dans un deuxième temps, on incorpore dans ce mélange l'élastomère thermoplastique et on continue à agiter toujours entre 100 à 130°C jusqu'à l'obtention d'un mélange homogène. Si nécessaire l'élastomère thermoplastique peut être mélangé et fondu avec une petite partie du plastifiant liquide avant incorporation pour faciliter son homogénéisation avec le mélange précédent.

Si nécessaire, on laisse refroidir à une température inférieure à 100°C et on incorpore alors tout composé susceptible de se dégrader à une température supérieure comme par exemple des adjuvants, des actifs liposolubles ou hydrosolubles tel un extrait sec d'Arnica dans l'exemple 1 et on agite jusqu'à obtention d'un mélange homogène.

L'émulsion solide est alors prête à être utilisée par exemple moulée, extrudée, ou bien encore incorporée dans un produit par enduction.

Les constituants utilisés pour la réalisation des émulsions solides décrites ci-après sont les suivants :
- PREPARATION 1 copolymère amphiphile
- KRATON D 1161^{®} SIS commercialisé par la société SHELL
- ONDINA 15® huile minérale commercialisée par la société SHELL
- GEMSEAL^{®} 60 mélange liquide d'hydrocarbures saturés commercialisé par la société TOTAL
- EAU
- EXTRAIT D'ARNICA actif hydrophile

Les quantités des différents constituants de ces émulsions solides exprimées en pourcentage en poids par rapport au poids total de l'émulsion solide sont rassemblées dans le tableau I.

**TABLEAU I**

| | EX.1 | EX. 2 | EX.3 | EX.4 | EX.5 | EX.6 |
|---|---|---|---|---|---|---|
| PREPARATION I | 0,1 | 2 | 0,5 | 1 | 1 | 0,25 |
| KRATON D 1161® | 10 | 2 | 9 | 5 | 10 | 10 |
| ONDINA 15® | | 44 | 66 | | | |
| GEMSEAL® 60 | 62,1 | | | 47 | 66 | 70 |
| EAU | 19,8 | 52 | 25 | 47 | 23 | 25 |
| EXTRAIT D'ARNICA | 8 | | | | | |

Les exemples du Tableau I illustrent les avantages de l'invention. On constate que l'incorporation de quantités très faibles de copolymères amphiphiles de l'ordre de 0,1 à 0,5% en poids par rapport au poids total de l'émulsion solide dans les exemples 1, 3 et 6, permet d'incorporer jusqu'à 20 à 25% d'eau à un gel hydrophobe à base de KRATON^{®}D et de plastifiant liquide.

De même, on a incorporé dans l'émulsion solide de l'exemple 1, 8% en poids d'un principe actif hydrophile, un extrait d'Arnica.

Si nécessaire, on peut enduire cette émulsion solide à chaud sur un support avec un adhésif périphérique et on obtient alors par exemple un patch à l'Arnica qui apporte fraîcheur et activité de l'Arnica pour traiter les coups et bosses. On peut éventuellement ne pas utiliser de support et réaliser directement un film ou une plaque contenant de l'Arnica et applicable sur la peau à l'aide d'une bande.

Enfin, dans les exemples 2 et 4, on constate que l'on obtient des émulsions solides à base de KRATON®D 1161 contenant jusqu'à 50% d'eau.

On a comparé par ailleurs les propriétés rhéologiques d'une émulsion solide selon l'invention, à celle d'une émulsion classique en testant leurs capacités de déformation sous contrainte.

On a ainsi testé une émulsion solide selon l'invention, celle de l'exemple 5, et une émulsion classique constituée par le produit commercialisé par la société URGO sous la dénomination URGO® brûlures.

Les conditions de test sont les suivantes :
- Matériel et équipement

On utilise un rhéomètre BOHLIN (type CS) à contrainte imposée. Le test est réalisé à la température de 25°C.

Le principe du test de relaxation de contrainte est le suivant :
Une contrainte suffisante pour déformer l'échantillon est appliquée pendant 60 secondes.

Cette contrainte est ensuite supprimée et l'on mesure pendant 120 secondes la déformation résultante y, exprimée en pourcentage, de l'échantillon.

L'appareil fournit directement des courbes qui illustrent les propriétés de déformation de l'échantillon.

Dans le cas présent, les deux produits ont des propriétés très différentes et l'on ne peut donc pas appliquer la même contrainte.

En effet, avec l'appareil utilisé sous une contrainte de 50 Pascals, la déformation de l'émulsion solide est insuffisante pour être mesurée alors que sous une contrainte de 100 Pascals, dans le cas de l'émulsion classique, la mesure ne peut être réalisée car une partie de l'émulsion est projetée sous l'effet de la rotation à l'extérieur des plateaux.

Les conditions de réalisation du test avec le rhéomètre BOHLIN sur chaque produit ont donc été adaptées pour tenir compte de ces éléments et avoir néanmoins des résultats comparables.
- Emulsion brûlure :
   L'échantillon à analyser (environ 1 gramme) est placé entre un plan inférieur stationnaire et un cône supérieur (diamètre 40 mm, angle 40 degrés).
- Emulsion solide de l'exemple 5 :
   L'échantillon à analyser (environ 1 gramme) est placé entre deux plateaux parallèles (diamètre 20 mm, distance entre les plateaux 1mm). On prend bien soin que l'échantillon déposé ne dépasse pas des plateaux.

La contrainte appliquée sur l'émulsion URGO® brûlures est donc de 50 Pascals et celle appliquée sur l'émulsion solide de l'exemple 5 est de 100 Pascals.

Cette différence de valeur de la contrainte appliquée illustre déjà en elle-même les avantages des émulsions solides selon l'invention par rapport aux émulsions classiques en termes de cohésion et d'élasticité.

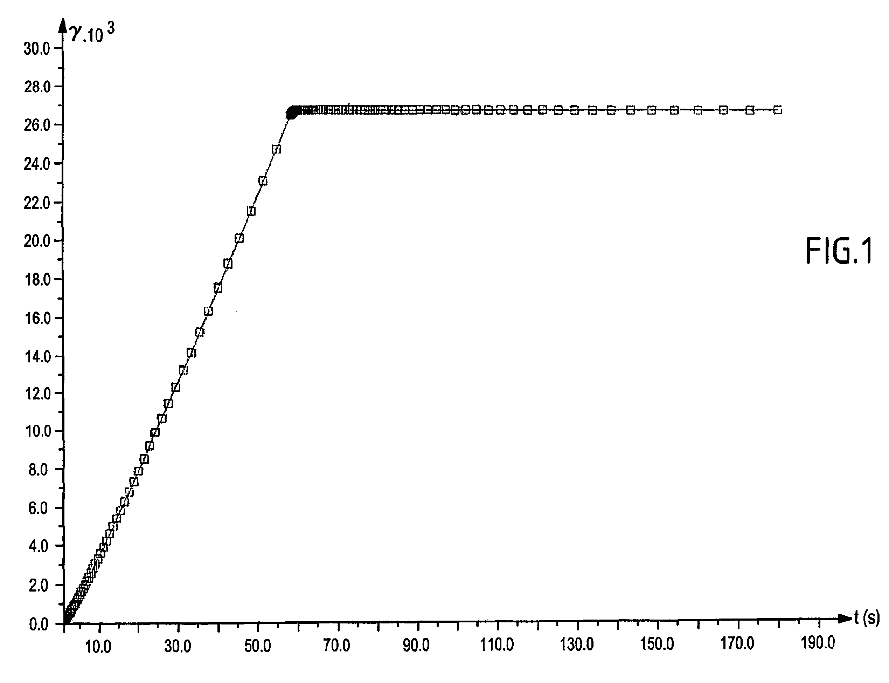

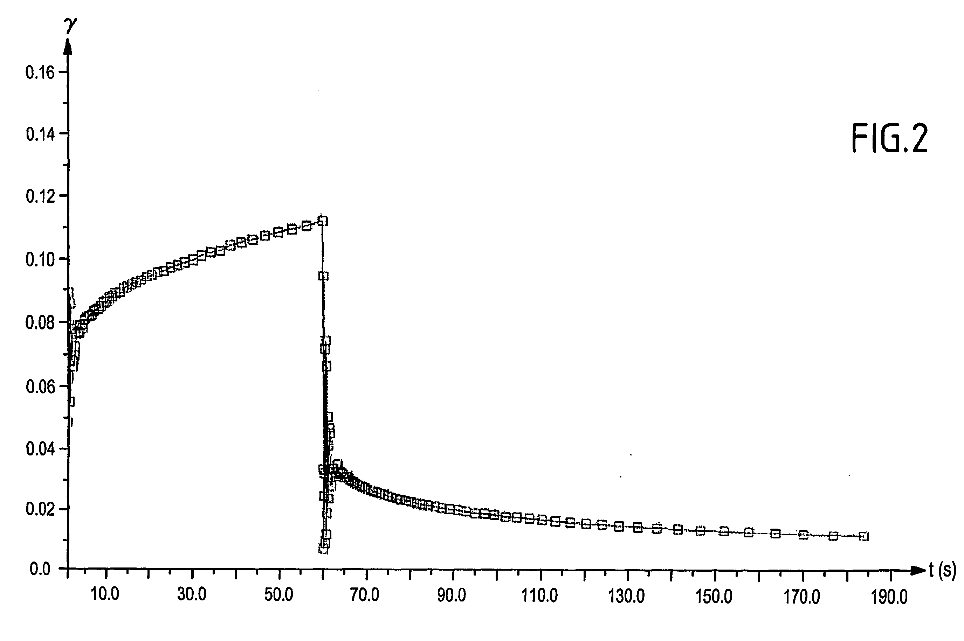

Les résultats obtenus sont illustrés sur les figures 1 et 2 qui concernent respectivement l'émulsion brûlure pour la figure 1 et l'émulsion solide de l'exemple 5 pour la figure 2.

Sur ces deux figures, on a porté en abscisse le temps exprimé en secondes et en ordonnée la déformation résultante y x 10³ exprimée en pourcentage sur la figure 1 et y exprimée en pourcentage sur la figure 2.

La comparaison des deux courbes démontre de façon très claire les différences en termes de propriétés rhéologiques, élasticité et cohésion, entre une émulsion classique et une émulsion solide selon l'invention.

Sur la figure 1, on voit que l'émulsion classique présente un caractère purement visqueux. Sous une contrainte donnée, la déformation augmente de façon linéaire avec le temps. Elle varie dans cet exemple de l'ordre de 26%.

Une fois la contrainte supprimée, il n'y a pas de retour à l'état initial. On obtient une droite horizontale, caractérisant une absence totale de caractère élastique et donc cohésif.

Au contraire, on voit sur la figure 2 que l'émulsion solide selon l'invention présente un comportement visco-élastique.

Sous une contrainte donnée, (le double de celle exercée sur l'émulsion classique), la déformation est beaucoup plus faible de l'ordre de 0,10%. En outre, et contrairement à l'émulsion classique, après la suppression de la contrainte, l'émulsion solide tend à retrouver son état initial (on n'observe pas de droite linéaire) et la déformation résultante y tend vers 0.

On a donc un produit élastique et donc cohésif.

Ce sont ces propriétés rhéologiques qui permettent d'obtenir des émulsions solides dont on peut régler la dureté en fournissant ainsi une large gamme de produits élastiques et cohésifs allant du gel au solide avec les avantages précédemment décrits.

## Revendications

1. Emulsion solide **caractérisée en ce qu'**elle comprend :
- un élastomère thermoplastique, choisi parmi les copolymères séquencés poly(styrène-oléfine-styrène), poly(styrène-oléfine) et leurs mélanges
- une phase huileuse constituée d'un plastifiant liquide
- une phase aqueuse et
- un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 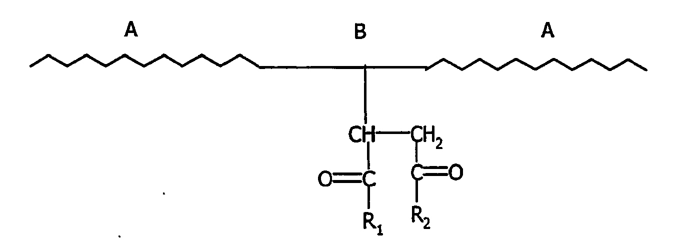
dans laquelle R₁ et R₂ identiques ou différents représentent un groupement hydrophile de masse molaire moyenne inférieure à 10 000, choisi parmi les groupes suivants :
CH₃-O-(CH₂-CH₂-O)ₙ;
HO-(CH₂-CH₂-O)ₙ; 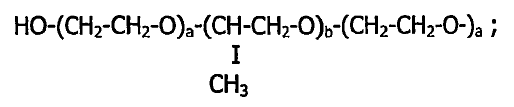
dans, lesquels n, a et b représentent un nombre entier.

2. Emulsion solide selon la revendication 1 **caractérisée en ce que** le copolymère amphiphile est un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 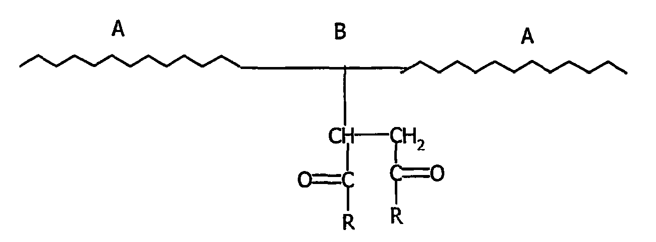 dans laquelle R représente un groupe CH₃-O-(CH₂-CH₂-O)ₙ de masse molaire moyenne égale à 2000 soit n=45.

3. Emulsion solide selon la revendication 2 **caractérisée en ce que** le copolymère amphiphile a une masse molaire moyenne mesurée par chromatographie par perméation de gel de l'ordre de 50 000 daltons.

4. Emulsion solide selon l'une des revendications précédentes **caractérisée en ce qu'**elle comprend de 0,05 à 20% et de préférence de 0,05 à 5% en poids de copolymère amphiphile par rapport au poids total de l'émulsion solide.

5. Emulsion solide selon l'une des revendications précédentes **caractérisée en ce que** le plastifiant liquide constituant la phase huileuse est une huile plastifiante ou un mélange liquide d'hydrocarbures saturés compatible avec la séquence centrale oléfine de l'élastomère thermoplastique et de préférence une huile plastifiante minérale et en particulier une huile de paraffine.

6. Emulsion solide selon la revendication 5 **caractérisée en ce qu'**elle comprend de 25 à 90% en poids et de préférence de 30 à 75% en poids de plastifiant liquide par rapport au poids total de l'émulsion solide.

7. Emulsion solide selon l'une des revendications précédentes **caractérisée en ce que** l'élastomère thermoplastique est un mélange de copolymère poly(styrène-oléfine-styrène) et de copolymère poly(styrène-oléfine)

8. Emulsion solide selon l'une des revendications précédentes **caractérisée en ce que** la séquence oléfine de l'élastomère thermoplastique précité est choisie parmi les séquences isoprène, butadiène, éthylène-butylène ou éthylène-propylène et de préférence est une séquence isoprène ou éthylène-butylène.

9. Emulsion solide selon l'une des revendications précédentes **caractérisée en ce que** l'élastomère thermoplastique est présent à une concentration de 2 à 20% en poids par rapport au poids total de l'émulsion solide.

10. Emulsion solide selon l'une des revendications précédentes **caractérisée en ce que** l'élastomère thermoplastique a une masse molaire moyenne supérieure à celle du copolymère amphiphile et de préférence supérieure ou égale à 100 000 daltons et de préférence **en ce qu'**il est présent à une concentration de 2 à 10% en poids par rapport au poids total de l'émulsion solide.

11. Emulsion solide selon l'une des revendications précédentes **caractérisée en ce qu'**elle comprend de 5 à 60% en poids d'eau et de préférence de 10 à 50% par rapport au poids total de l'émulsion solide.

12. Emulsion solide notamment utilisable sur la peau, la plaie ou les muqueuses **caractérisée en ce qu'**elle comprend :
a. 2 à 10 parties en poids d'un élastomère thermoplastique de masse molaire moyenne supérieure ou égale à 100 000 daltons
b. 30 à 75 parties en poids de plastifiant liquide
c. 2 à 50 parties en poids d'eau et
d. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 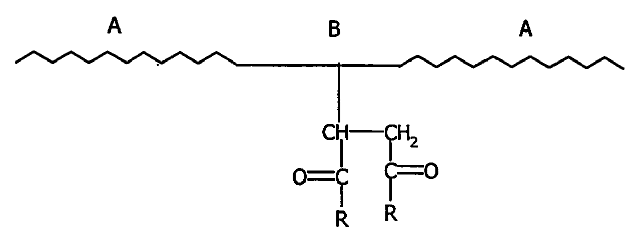
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

13. Utilisation d'une émulsion solide selon l'une des revendications 1 à 12, pour la fabrication d'un produit destiné à des fins médicales, dermatologiques, cosmétologiques ou pharmaceutiques ou chirurgicales.

14. Utilisation d'une émulsion solide selon l'une des revendications 1 à 12, pour la fabrication d'un produit à appliquer sur la peau, la plaie ou les muqueuses

15. Utilisation d'une émulsion solide selon l'une des revendications 13 ou 14 **caractérisée en ce que** le produit est un pansement pour le traitement ou la protection de la plaie, de l'ampoule, des brûlures, ou des lésions dermo-épidermiques superficielles, un patch pour la délivrance d'actifs par voie topique ou systémique, un produit de soin, de nettoyage ou de protection de la peau ou des muqueuses, une électrode, ou une plaque ou un film conformable.

## Claims

1. A solid emulsion, **characterised in that** it comprises :
- a thermoplastic elastomer, which is selected from the block copolymers poly(styrene-olefin-styrene), poly(styrene-olefin) and their mixtures,
- an oily phase, which is constituted of a liquid plasticizer,
- an aqueous phase, and
- an amphiphilic ABA type block copolymer comprising two terminal thermoplastic poly(styrene) blocks A and one central elastomeric block B, in which the central block B is a poly(ethylene-butylene) sequence comprising grafted hydrophilic groups, it being possible for said amphiphilic copolymer ABA to be represented schematically by the following structure : 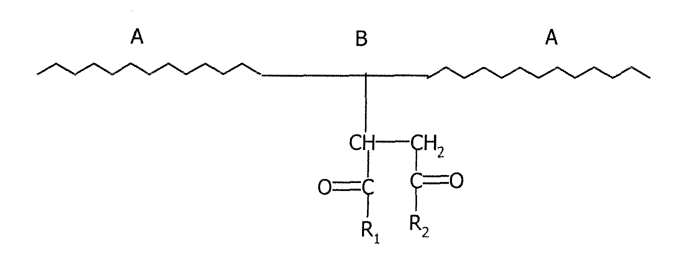
in which R₁ and R₂, which are identical or different, represent a hydrophilic group of average molar mass of less than 10,000, selected from the following groups :
CH₃-O-(CH₂-CH₂-O)n ;
HO-(CH₂-CH₂-O)ₙ ; 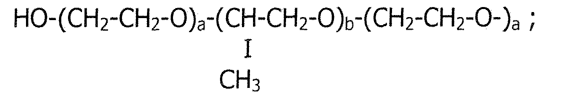
in which n, a and b represent an integer.

2. The solid emulsion according to claim 1, **characterised in that** the amphiphilic copolymer is an amphiphilic ABA type block copolymer comprising two terminal thermoplastic poly(styrene) blocks A and one central elastomeric block B, in which the central block B is a poly(ethylene-butylene) sequence comprising grafted hydrophilic groups, it being possible for said amphiphilic copolymer ABA to be represented schematically by the following structure : 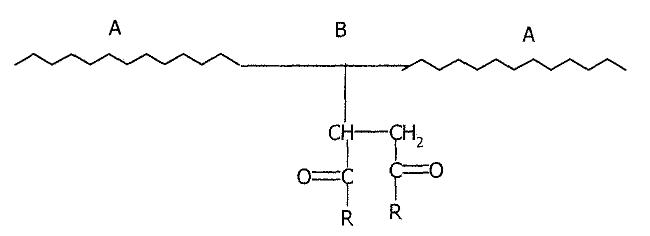 in which R represents a CH₃-O-(CH₂-CH₂-O)ₙ group of average molar mass equal to 2000, *i.e.* n=45.

3. The solid emulsion according to claim 2, **characterised in that** the amphiphilic copolymer has an average molar mass measured by gel permeation chromatography of the order of 50,000 daltons.

4. The solid emulsion according to one of the preceding claims, **characterised in that** it comprises 0.05 to 20%, preferably 0.05 to 5%, by weight, of amphiphilic copolymer with respect to the total weight of the solid emulsion.

5. The solid emulsion according to one of the preceding claims, **characterised in that** the liquid plasticizer constituting the oily phase is a plasticizing oil or a liquid mixture of saturated hydrocarbons, which is compatible with the central olefin sequence of the thermoplastic elastomer, preferably a plasticizing mineral oil, particularly a liquid paraffin.

6. The solid emulsion according to claim 5, **characterised in that** it comprises 25 to 90% by weight, preferably 30 to 75% by weight, of liquid plasticizer with respect to the total weight of the solid emulsion.

7. The solid emulsion according to one of the preceding claims, **characterised in that** the thermoplastic elastomer is a mixture of poly(styrene-olefin-styrene) copolymer and of poly(styrene-olefin) copolymer.

8. The solid emulsion according to one of the preceding claims, **characterised in that** the olefin sequence of the thermoplastic elastomer cited above is selected from the sequences : isoprene, butadiene, ethylene-butylene or ethylene-propylene, and is preferably an isoprene or ethylene-butylene sequence.

9. The solid emulsion according to one of the preceding claims, **characterised in that** the thermoplastic elastomer is present at a concentration of 2 to 20% by weight with respect to the total weight of the solid emulsion.

10. The solid emulsion according to one of the preceding claims, **characterised in that** the thermoplastic elastomer has an average molar mass greater than that of the amphiphilic copolymer, preferably greater than or equal to 100,000 daltons, and preferably **in that** it is present at a concentration of 2 to 10% by weight with respect to the total weight of the solid emulsion.

11. The solid emulsion according to one of the preceding claims, **characterised in that** it comprises 5 to 60% by weight of water, preferably 10 to 50%, with respect to the total weight of the solid emulsion.

12. A solid emulsion usable notably on the skin, a wound or the mucous membranes, **characterised in that** it comprises :
a. 2 to 10 parts by weight of a thermoplastic elastomer of average molar mass greater than or equal to 100,000 daltons,
b. 30 to 75 parts by weight of liquid plasticizer,
c. 2 to 50 parts by weight of water, and
d. 0.05 to 5 parts by weight of an amphiphilic ABA type block copolymer comprising two terminal thermoplastic poly(styrene) blocks A and one central elastomeric block B, in which the central block B is a poly(ethylene-butylene) sequence comprising grafted hydrophilic groups, it being possible for said amphiphilic copolymer ABA to be represented schematically by the following structure : 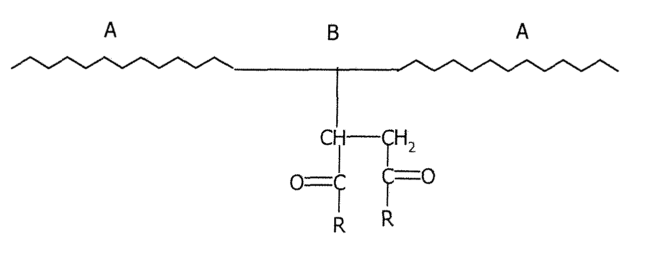
in which R represents a CH₃-O-(CH₂-CN₂-O)ₙ group of average molar mass equal to 2000, *i.e.* n=45, and which has an average molar mass measured by gel permeation chromatography of 50,000 daltons.

13. Use of a solid emulsion according to one of claims 1 to 12 for the manufacture of a product intended for medical, dermatological, cosmetological or pharmaceutical or surgical purposes.

14. Use of a solid emulsion according to one of claims 1 to 12 for the manufacture of a product to be applied on the skin, a wound or the mucous membranes.

15. Use of a solid emulsion according to one of claims 13 or 14, **characterised in that** the product is a dressing for treating or protecting a wound, a blister, burns, or superficial dermo-epidermic lesions, a patch for delivering actives via the topical or systemic route, a product for the care, the cleansing or the protection of the skin or of the mucous membranes, an electrode, or a sheet or a shapable film.

## Patentansprüche

1. Feste Emulsion, **dadurch gekennzeichnet, dass** sie umfasst:
- ein thermoplastisches Elastomer, das aus den sequenzierten Copolymeren Poly(Styrol-Olefin-Styrol), Poly(Styrol-Olefin) und deren Gemischen gewählt ist
- eine ölige Phase, die aus einem flüssigen Weichmacher gebildet ist
- eine wässrige Phase, und
- ein amphiphiles Block-Copolymer der Art ABA mit zwei terminalen thermoplastischen Blöcken A Poly(styrol) und einem mittleren Elastomer-Block B, in welchem der mittlere Block B eine Sequenz Poly(Ethylen-Butylen) ist, die gepfropfte hydrophile Gruppen aufweist, wobei das amphiphile Copolymer ABA schematisch durch die folgende Struktur dargestellt werden kann: 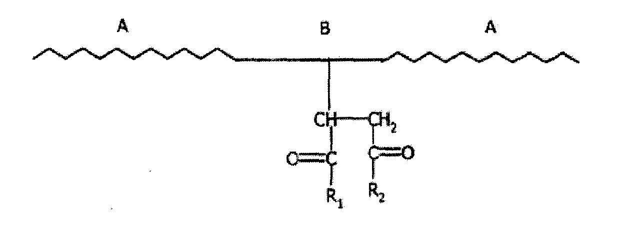
in welcher R₁ und R₂, identisch oder verschieden, eine hydrophile Gruppierung mit einer mittleren Molmasse kleiner 10.000 darstellen, die aus den folgenden Gruppen gewählt wird:
CH₃-O-(CH₂-CH₂-O)ₙ ;
HO-(CH₂-CH₂-O)ₙ ; 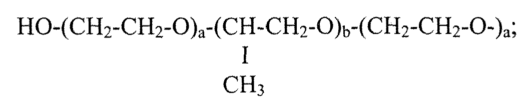
in welchen n, a und b eine ganze Zahl darstellen.

2. Feste Emulsion gemäß Anspruch 1, **dadurch gekennzeichnet, dass** das amphiphile Copolymer ein amphiphiles Block-Copolymer der Art ABA mit zwei terminalen thermoplastischen Blöcken A Poly(Styrol) und einem mittleren Elastomer-Block B, in welchem der mittlere Block B eine Sequenz Poly(Ethylen-Butylen) ist, die gepfropfte hydrophile Gruppen aufweist, wobei das amphiphile Copolymer ABA schematisch durch die folgende Struktur dargestellt werden kann: 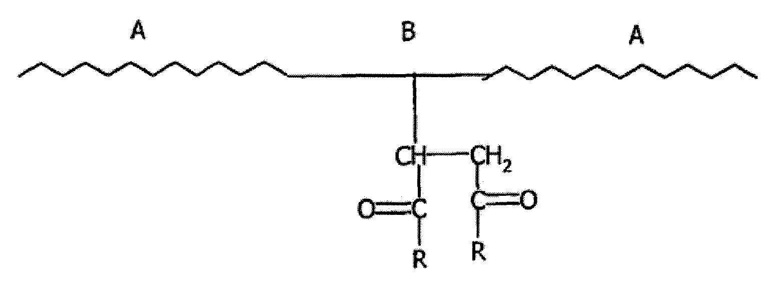 in welcher R eine Gruppe CH₃-O-(CH₂-CH₂-O)ₙ mit einer mittleren Molmasse gleich 2.000, wobei n=45 ist, darstellt.

3. Feste Emulsion gemäß Anspruch 2, **dadurch gekennzeichnet, dass** das amphiphile Copolymer eine mittlere Molmasse aufweist, die durch Gel-Permeations-Chromatographie gemessen wurde und im Bereich von 50.000 Dalton liegt.

4. Feste Emulsion gemäß einem der vorausgegangenen Ansprüche, **dadurch gekennzeichnet, dass** sie 0,05 bis 20 Gew.-%, und vorzugsweise 0,05 bis 5 Gew.-%, an amphiphilem Copolymer bezogen auf das Gesamtgewicht der festen Emulsion aufweist.

5. Feste Emulsion gemäß einem der vorausgegangenen Ansprüche, **dadurch gekennzeichnet, dass** der flüssige Weichmacher, der die ölige Phase bildet, ein Weichmacher-Öl oder ein flüssiges Gemisch gesättigter Kohlenwasserstoffe ist, das mit der mittleren Olefin-Sequenz des thermoplastischen Elastomers kompatibel ist, und vorzugsweise ein Weichmacher-Mineralöl und insbesondere ein Paraffin-Öl ist.

6. Feste Emulsion gemäß Anspruch 5, **dadurch gekennzeichnet, dass** sie 25 bis 90 Gew.-%, und vorzugsweise 30 bis 75 Gew.-%, des flüssigen Weichmachers bezogen auf das Gesamtgewicht der festen Emulsion aufweist.

7. Feste Emulsion gemäß einem der vorausgegangenen Ansprüche, **dadurch gekennzeichnet, dass** das thermoplastische Polymer ein Gemisch aus Copolymer Poly(Styrol-Olefin-Styrol) und Copolymer Poly(Styrol-Olefin) ist.

8. Feste Emulsion gemäß einem der vorausgegangenen Ansprüche, **dadurch gekennzeichnet, dass** die Olefin-Sequenz des thermoplastischen Elastomers aus den Sequenzen Isopren, Butadien, Ethylen-Butylen oder Ethylen-Propylen gewählt wird und vorzugsweise eine Sequenz Isopren oder Ethylen-Butylen ist.

9. Feste Emulsion gemäß einem der vorausgegangenen Ansprüche, **dadurch gekennzeichnet, dass** das thermoplastische Elastomer in einer Konzentration von 2 bis 20 Gew.-% bezogen auf das Gesamtgewicht der festen Emulsion vorhanden ist.

10. Feste Emulsion gemäß einem der vorausgegangenen Ansprüche, **dadurch gekennzeichnet, dass** das thermoplastische Elastomer eine mittlere Molmasse aufweist, die größer ist als die des amphiphilen Copolymers, und vorzugsweise gleich oder größer ist als 100.000 Dalton, und vorzugsweise **dadurch**, dass sie in einer Konzentration von 2 bis 10 Gew.-% bezogen auf das Gesamtgewicht der festen Emulsion vorhanden ist.

11. Feste Emulsion gemäß einem der vorausgegangenen Ansprüche, **dadurch gekennzeichnet, dass** sie 5 bis 60 Gew.-% Wasser, und vorzugsweise 10 bis 50%, bezogen auf das Gesamtgewicht der festen Emulsion aufweist.

12. Feste Emulsion, die insbesondere auf der Haut, auf Wunden oder Schleimhäuten anwendbar ist, **dadurch gekennzeichnet, dass** sie umfasst:
a. 2 bis 10 Gew.-Teile eines thermoplastischen Elastomers mit einer Molmasse größer oder gleich 100.000 Dalton
b. 30 bis 75 Gew.-Teile eines flüssigen Weichmachers
c. 2 bis 50 Gew.-Teile Wasser und
d. 0,05 bis 5 Gew.-Teile eines amphiphilen Block-Copolymers der Art ABA mit zwei terminalen thermoplastischen Blöcken A Poly(Styrol) und einem mittleren Elastomer-Block B, in welchem der mittlere Block B eine Sequenz Poly(Ethylen-Butylen) ist, die gepfropfte hydrophile Gruppen aufweist, wobei das amphiphile Copolymer ABA schematisch durch die folgende Struktur dargestellt werden kann: 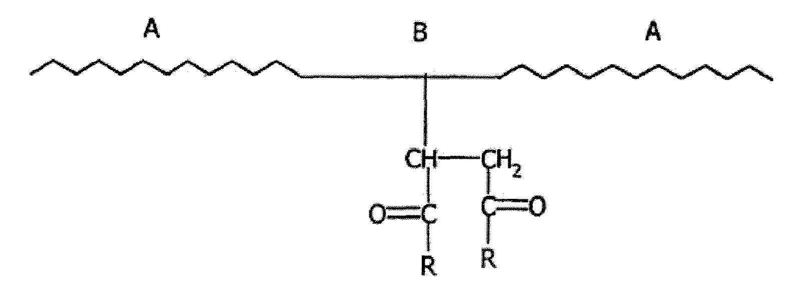
in welcher R eine Gruppe CH₃-O-(CH₂-CH₂-O)ₙ mit einer mittleren Molmasse gleich 2.000, wobei n=45 ist, darstellt, und die eine mittlere Molmasse aufweist, die durch Gel-Permeations-Chromatographie gemessen wurde und im Bereich von 50.000 Dalton liegt.

13. Verwendung einer festen Emulsion gemäß einem der Ansprüche 1 bis 12 für die Herstellung eines Produkts, das für medizinische, dermatologische, kosmetische oder pharmazeutische oder chirurgische Zwecke bestimmt ist.

14. Verwendung einer festen Emulsion gemäß einem der Ansprüche 1 bis 12 für die Herstellung eines Produkts, das auf die Haut, Wunden oder Schleimhäute aufgebracht wird.

15. Verwendung einer festen Emulsion gemäß einem der Ansprüche 13 oder 14, **dadurch gekennzeichnet, dass** das Produkt ein Verband für die Behandlung oder den Schutz von Wunden, Blasen, Verbrennungen oder oberflächlichen dermo-epidermalen Läsionen, ein Pflaster für das Liefern von Wirkstoffen auf topischem oder systemischem Weg, ein Pflegeprodukt, ein Reinigungsprodukt oder ein Produkt zum Schutz der Haut und der Schleimhäute, eine Elektrode oder eine Anode oder ein anpassbarer Film ist.
